# EUROPEAN PATENT APPLICATION

(11) **EP 2 343 684 A1**
(43) Date of publication of application: **13.07.2011**
(21) Application number: 09821781.3
(22) Date of filing: 19.10.2009
(51) Int. Cl.: G06Q 50/00

(54) **INSPECTION MANAGING DEVICE**

(30) Priority: 23.10.2008 JP 2008273515
(71) Applicant: Olympus Medical Systems Corp., Tokyo 151-0072 (JP)
(72) Inventor: INABA, Hirofumi, Tokyo 151-0072 (JP); YASUDA, Hideki, Tokyo 151-0072 (JP); SUZUKI, Hitoshi, Tokyo 151-0072 (JP); NAKAHARA, Tetsuyuki, Tokyo 151-0072 (JP); YAMAGATA, Fumio, Tokyo 151-0072 (JP); NOMURA, Tadakuni, Tokyo 151-0072 (JP); ISHIZUKA, Tatsuya, Tokyo 151-0072 (JP); KOBAYASHI, Makoto, Tokyo 151-0072 (JP); KURIYAMA, Saori, Tokyo 151-0072 (JP); YADA, Kohei, Tokyo 151-0072 (JP)
(74) Representative: Gunzelmann, Rainer
(86) International application number: PCT/JP2009/005467
(87) International publication number: WO 2010/047081

(57) **Abstract**

An inspection schedule holding unit (10) holds an inspection schedule containing a plurality of inspections. An operational situation acquiring unit (20) acquires the operational situations of a cleaning device (210) for cleaning a scope to be used in the inspections. In accordance with an operational situations acquired by the operational situation acquiring unit (20), a reschedule unit (30) changes an inspection starting time of at least one of the inspections contained in the inspection schedule. The reschedule unit (30) specifies the cleaning ending time of the used scope from the cleaning starting time acquired by the operational situation acquiring unit (20), and decides whether or not the cleaned scope exists at the inspection starting time of each inspection, in accordance with the cleaning ending time and the inspection starting time of each inspection. In the case of the nonexistence, the inspection starting time of the inspection may be changed to the instant, at which the cleaned scope exists, or later.

## Description

### [Technical Field]

The present invention relates to an examination management apparatus for managing a schedule of examinations for which endoscopes are used.

### [Background Art]

Endoscopes (hereinafter, simply referred to as "scopes") used for endoscopic examinations need to be washed every time the examinations are finished. Therefore, an apparatus for washing (hereinafter, referred to as a washing apparatus), which is used for washing used scopes, is installed in a medical facility. When there are many endoscopic examinations to be performed in one day, scopes are washed and repeatedly used for the examinations.

### RELATED ART DOCUMENTS

### PATENT DOCUMENTS

[Patent document No. 1] JP 2004-102869
[Patent document No. 2] JP 2005-128624

### [Disclosure of Invention]

A standard examination schedule for a day is determined in consideration of examination time, unoccupied time between examinations, etc. However, actual examinations are not always performed as scheduled in the examination schedule. Taking more time for an examination than that which is scheduled is a typical example of one of the causes why examinations are not being performed as scheduled. However, another cause can also be not being able to prepare an already-washed scope at the scheduled start time of an examination due to the delay in washing a used scope.

A used scope needs to be set in a washing apparatus by the staff. Thus, the delay in setting a scope will result in an excess of used scopes and eventually in a shortage of already-washed scopes. It is ideal to wash the scopes used for an examination without any delay after the examination has been completed; however, it is often difficult in most medical facilities, where manpower tends to be in short supply.

In the past, the start time of an examination often had to be delayed due to a lack of already-washed scopes at the scheduled start time of the examination. The patient or medical staff had to change the schedule without notice when the medical staff became aware, at the last minute, before the start time of an examination, that a scope could not be washed in time. If the patient is notified, in it is possible for a patient to visit a hospital at the rescheduled start time of the examination.

The present invention has been made in view of problems such as described above, and a purpose thereof is to provide an examination management apparatus for detecting of the necessity of changing an examination schedule as early as possible and thus for an early change of the examination schedule.

### [Means for Solving the Problem]

An examination management apparatus according to one embodiment of the present invention comprises: an examination schedule memory unit operative to store an examination schedule including a plurality of examinations; an operation-status acquisition unit operative to acquire operation-status information that indicates the operation status of a washing apparatus for washing a scope used in the examination; and a rescheduling unit operative to change the start time of at least one examination included in the examination schedule in accordance with the operation-status information acquired by the operation-status acquisition unit.

Another embodiment of the present invention also relates to an examination management apparatus. The apparatus comprises: a standard-time memory unit operative to store, according to examination time, the respective standard examination; a time acquisition unit operative to acquire the start time and the completion time of each process of the examination from at least one device installed in the examination room where the examination is performed; and a display control unit operative to display, on a display unit of the terminal apparatus installed in the examination room, a first display content, which indicates the progress status to be achieved of the examination based on the standard time that is based on the examination time, and a second display content, which indicates the actual progress status based on the start time and the completion time of each of the processes of the examination acquired by the time acquisition unit.

Optional combinations of the aforementioned constituting elements and implementations of the invention in the form of methods, apparatuses, systems, recording mediums, and computer programs may also be practiced as additional modes of the present invention.

### [Advantageous Effects]

The present invention allows for detecting of the necessity of changing an examination schedule as early as possible and thus for an early change of the examination schedule.

Fig. 1 is a view of the whole configuration of an examination management system according to the embodiment 1 of the present invention;
Fig. 2 is a functional block diagram showing the configuration of an examination management apparatus according to the embodiment 1;
Fig. 3 is a view showing an example of the transition of both an examination schedule and the number of available scopes according to the embodiment 1 (no delay in washing-start time);
Fig. 4 is a view showing an example of the transition of both an examination schedule and the number of available scopes (prior to any change in the start time of the examination) according to the embodiment 1 (some delay in washing-start time);
Fig. 5 is a view showing an example of the transition of both an examination schedule and the number of available scopes (after any change in the start time of the examination) according to the embodiment 1 (some delay in washing-start time);
Fig. 6 is a flowchart showing a reschedule process of an examination management system according to the embodiment 1;
Fig. 7 is a functional block diagram showing the configuration of an examination management apparatus
Fig. 8 is a diagram showing an example of a screen display-controlled by a display control unit according to the embodiment 2 (in the middle of an examination);
Fig. 9 is a diagram showing an example of a screen display-controlled by a display control unit according to the embodiment 2 (after an examination); and
Fig. 10 is a diagram showing another example of a screen display-controlled by a display control unit according to the embodiment 2.

### [Best Mode for Carrying Out the Invention]

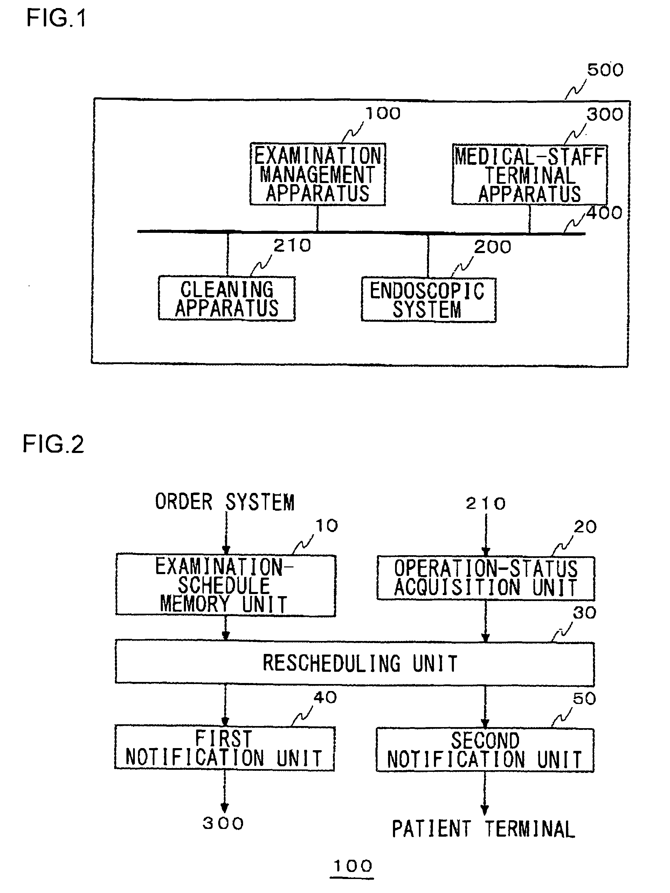

Fig. 1 is a view of the whole configuration of an examination management system 500 according to the embodiment 1 of the present invention. The examination management system 500 is provided with an examination management apparatus 100, an endoscopic system 200, a washing apparatus 210, and a medical-staff terminal apparatus 300. These systems and apparatuses are connected to one another via a network 400 such as intranet.

The examination management apparatus 100 manages an examination schedule and particularly the examination schedule of an endoscopic examination in the present embodiment. The examination management apparatus 100 will be described in detail hereinafter.

The endoscopic system 200 includes an endoscopic examination-room terminal apparatus (not shown) that displays images captured by a scope on a monitor and stores patient information, diagnostic information, captured images, etc. The endoscopic system 200 allows for the examination management apparatus 100 to be notified via a network 400 of the start time and the completion time of each examination. As the start time of each examination, the time when a scope is inserted into the body of a patient may be detected automatically by using, for example, a sensor, or the time when the medical staff operates the above examination-room terminal apparatus while indicating the start of the examination may be used. As the completion time of each examination, the time when a scope is pulled out from the body of a patient may be detected automatically by using, for example, a sensor or the time when the medical staff operates the above examination-room terminal apparatus while indicating the completion of the examination may be used.

The washing apparatus 210 washes and sterilizes scopes that have been used for examinations. Unique identification information for self-identification is provided to each scope. For example, an IC tag using the RFID (Radio Frequency IDentification) method is attached to the cord of a scope. The washing apparatus 210 has a function of reading out the identification information of a scope that is set therein. Upon the start of washing the notice, which includes the identification information of the scope and the washing start time, to the examination management apparatus 100 via the network 400. The washing apparatus 210 may retrieve the identification information for the unique identification of a nurse or of a worker, who is exclusively in charge of a washing task, from an IC tag the nurse or the worker is wearing and may include the identification information in the above-stated washing-start notice.

During the washing of the scope, the washing apparatus 210 may transmit a washing-status notice, which includes the identification information of the scope and the remaining washing time or the scheduled washing completion time, to the examination management apparatus 100. Upon the completion of washing the scope, the washing apparatus 210 may transmit a washing-completion notice, which includes the identification information of the scope and the washing completion time, to the examination management apparatus 100. The washing-status notice and the washing-completion notice may be transmitted to the medical-staff terminal apparatus 300 carried by a nurse or a worker in charge of a washing task.

That described above has been based on the premise that the washing apparatus 210 has the function of communication. However, if the washing apparatus 210 does the washing-status notice, and the washing-completion notice may be transmitted from the medical-staff terminal apparatus 300 by a nurse or by a worker in charge of a washing task manually entering the notices into the medical-staff terminal apparatus 300 carried by the nurse or the worker. Also, the notices may be manually entered to and then transmitted from a PC in the room where the washing apparatus 210 is placed. Although Fig. 1 shows only one washing apparatus 210, there may be a plurality of washing apparatuses 210. A plurality of washing apparatuses 210 are often installed in a large medical facility. When a plurality of washing apparatuses 210 are installed, the washing-start notice, the washing-status notice, and the washing-completion notice are transmitted from each of the washing apparatuses 210.

The medical-staff terminal apparatus 300 is a terminal that can be referred to by, for example, a doctor, a nurse, or a worker exclusively in charge of a washing task. A portable terminal apparatus such as a PC placed in, for example, a treatment room, an examination room, or a nurse station and a PDA (Personal Digital Assistant), which a nurse carries, represents the medical-staff terminal apparatus 300. This portable terminal apparatus can communicate wirelessly with the examination management apparatus 100 via an access point on the network 400.

Fig. 2 is a functional block diagram showing the according to the embodiment 1. The examination management apparatus 100 includes an examination-schedule memory unit 10, an operation-status acquisition unit 20, a rescheduling unit 30, a first notification unit 40, and a second notification unit 50.

The configuration of the examination management apparatus 100 is implemented in hardware by any CPU of a computer, memory, or other LSI's, and in software by a program or the like loaded into the memory. Fig. 2 depicts functional blocks implemented by the cooperation of hardware and software. Thus, a person skilled in the art should appreciate that there are many ways of accomplishing these functional blocks in various forms in accordance with the components of hardware only, software only, or the combination of both.

The examination-schedule memory unit 10 stores an examination schedule including a plurality of examinations. More specifically, the examination-schedule memory unit 10 stores the start time and the completion time of an examination included in the examination schedule. The examination schedule is generated by an examination order system (not shown) based on an examination order by a doctor. However, since the subject invention is not focused on a method of generating an examination schedule, the subject invention is pursued based on the premise that an examination schedule memory unit 10. When multiple examination rooms are established, multiple examinations can be concurrently performed. A specific example of an examination schedule will be described in detail hereinafter. The examination schedule is limited to those examinations where a scope is used.

The operation-status acquisition unit 20 acquires operation-status information that indicates the operation status of the washing apparatus 210 that washes a scope used for an examination. For example, the operation-status acquisition unit 20 acquires a washing-start time for starting the washing of a used scope generated upon the completion of each examination by the washing apparatus 210. The operation-status acquisition unit 20 can acquire the washing-start time from the washing-start notice.

The rescheduling unit 30 changes the start time of at least one examination listed in an examination schedule in accordance with the operation-status information acquired by the operation-status acquisition unit 20. More specifically, the rescheduling unit 30 specifies the washing-completion time of a used scope based on the washing-start time acquired by the operation-status acquisition unit 20. The washing time of a scope is known in the washing apparatus 210, and the rescheduling unit 30 can thus specify, as the washing-completion time, the time obtained by adding the

Based on the specified washing-completion time and the start time of each examination that is specified based on the examination schedule, the rescheduling unit 30 determines whether or not an already-washed scope will be available at the start time of each examination. When it is determined that an already-washed scope will be available, the rescheduling unit 30 does not change the examination schedule. When it is determined that no already-washed scopes will be available, the rescheduling unit 30 changes the start time of the examination to be after the time at which an already-washed scope will be available upon the completion of the washing.

A detailed description is now given in the following. By using, as an initial value, the number of already-washed scopes at the start of a simulation, the rescheduling unit 30 simulates the transition of the number of available scopes in which the number decreases by one upon the start of an examination after the start time of the simulation and in which the number increases by one upon the completion of the washing of a used scope. In this manner, the rescheduling unit 30 predicts whether or not an already-washed scope will be available at the start time of each examination included in the examination schedule after the start time of the simulation and predicts, when there will be no already-washed scope available, the time at which an which an already-washed scope will become available can be predicted based on the washing-completion time, in the washing apparatus 210, of a scope.

When the examination schedule is changed by the rescheduling unit 30, the first notification unit 40 transmits to the medical-staff terminal apparatus 300 the examination schedule as changed via the network 400. The examination schedule as changed may be displayed on an electrical bulletin board placed in a patient waiting room.

The second notification unit 50 transmits schedule-change information including at least the changed start time of an examination to a patient terminal apparatus that can be referred to by the patient scheduled for the examination whose start time has been changed by the rescheduling unit 30. A PC installed at a patient's house, a cell phone a patient carries, etc., all correspond to the patient terminal apparatus. The second notification unit 50 transmits the schedule-change information to the patient terminal apparatus via e-mail, etc.

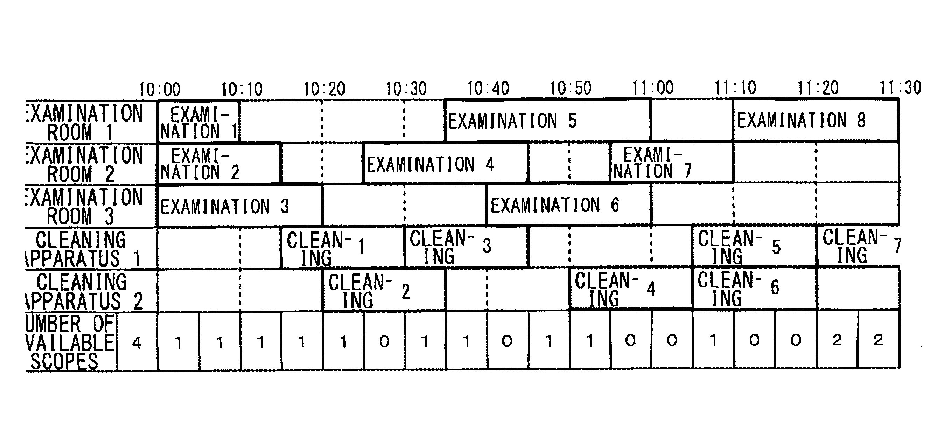

A detailed description will be made hereinafter by using specific examples of a rescheduling process performed by the rescheduling unit 30. Fig. 3 is a view showing an example of the transition of both an examination schedule and the number of available scopes according to the embodiment 1 (no delay in washing-start time). A detailed three examination rooms (examination rooms 1-3), two washing apparatuses (washing apparatuses 1 and 2), and four scopes in a medical facility.

A used scope needs to be hand-washed for about two minutes before the used scope is set into a washing apparatus. In addition, in consideration of, for example, the time required for taking out the used scope from the endoscopic apparatus and the time required for setting the used scope in the washing apparatus, it is assumed that the time required from the completion time of an examination to the washing-start time of the used scope is five minutes. This is the time required when a nurse or a worker in charge of the washing task performs the washing work in an ideal manner. Technically, after the washing-completion time, time is required for taking out a scope from the washing apparatus before the scope becomes available for an examination; however, the time required for a scope to become available after the washing-completion time is determined to be zero minutes to simplify the explanation. The washing time required for one wash is 15 minutes for each of washing apparatuses 1 and 2. There is a type of washing apparatus that can wash a plurality of scopes in one wash; however it is assumed that a washing apparatus is used to wash one scope per wash in order to simplify the explanation in the following. between 10:00 to 11:30 in the examination schedule of the day. Fig. 3 shows an example where a nurse or a worker in charge of the washing task performs the washing work in an ideal manner. At 10:00, examinations 1-3 start in the examination rooms 1-3, respectively. As of 10:00, the washing apparatuses 1 and 2 are ready and waiting. The number of available scopes right before 10:00 is four. The examinations 1-3 start at 10:00. Since one scope is used for each examination, the number of available scopes after 10:00 is one.

An examination 1 performed in the examination room 1 starts at 10:00 and lasts for 10 minutes, an examination 2 performed in the examination room 2 starts at 10:00 and lasts for 15 minutes, and an examination 3 performed in the examination room 3 starts at 10:00 and lasts for 20 minutes. In other words, the examination completion time of the examination 1 is 10:10, the examination completion time of the examination 2 is 10:15, and the examination completion time of the examination 3 is 10:20. Wash 1 for a scope that has finished being used for the examination 1 at 10:10 starts after five minutes, which is at 10:15, in the washing apparatus 1. Wash 2 for a scope that has finished being used for the examination 2 at 10:15 starts after five minutes, which is at 10:20, in the washing apparatus 2.
the washing apparatuses 1 and 2 are in the middle of washing at 10:25, which is five minutes after the completion of the examination 3, the scope used for the examination 3 cannot be washed starting at 10:25. Since the completion time of the wash 1 in the washing apparatus 1 is 10:30 and the completion time of the wash 2 in the washing apparatus 2 is 10:35, the scope used for the examination 3 is placed in a queue for the washing apparatus 1, which finishes washing first.

An examination 4 performed in the examination room 2 starts at 10:25 and lasts for 20 minutes. The examination 4 starts at 10:25. Since one scope is used for the examination, the number of available scopes after 10:25 decreases by one and therefore becomes zero. The wash 1 finishes at 10:30, and the number of available scopes after 10:30 increases by one and therefore becomes one. At 10:30, the washing apparatus 1 starts a wash 3 for the scope used for the examination 3, which had been placed in the queue for the washing apparatus 1.

The wash 2 finishes at 10:35, and the number of available scopes then increases by one. An examination 5 performed in the examination room 1 starts at 10:35 and lasts for 25 minutes. One scope is used for the examination. Thus, the number of available scopes decreases by one. The number of available scopes increases by one and decreases by one and therefore becomes one after 10:35. An examination 6 for 20 minutes. Since one scope is used for the examination, the number of available scopes after 10:40 decreases by one and therefore becomes zero.

The examination 4 finishes at 10:45. The wash 3 finishes at 10:45, and the number of available scopes after 10:45 increases by one and therefore becomes one. Wash 4 for the scope that has finished being used for the examination 4 at 10:45 starts after five minutes, which is at 10:50, in the washing apparatus 2. The scope may obviously be washed in the washing apparatus 1.

An examination 7 performed in the examination room 2 starts at 10:55 and lasts for 15 minutes. The examination 7 starts at 10:55. Since one scope is used for the examination, the number of available scopes after 10:55 decreases by one and therefore becomes zero. The examination 5 and the examination 6 finish at 11:00. Wash 5 for the scope that has finished being used for the examination 1 at 11:00 starts after five minutes, which is at 11:05, in the washing apparatus 1. Wash 7 for the scope that has finished being used for the examination 6 at 11:00 starts after five minutes, which is at 11:05, in the washing apparatus 2.

The examination 7 finishes at 11:10. Since both the washing apparatuses 1 and 2 are in the middle of being washed at 11:15, which is five minutes after the completion of the examination 7, the scope used for the examination 7 the queue for the washing apparatus 1. An examination 8 performed in the examination room 1 starts at 11:10 and lasts for 20 minutes. The examination 8 starts at 11:10. Since one scope is used for the examination, the number of available scopes after 11:10 decreases by one and therefore becomes zero. The wash 5 and the wash 6 finish at 11:20, and the number of available scopes increases by two and decreases by two and therefore becomes two after 11:20. The washing apparatus 1 starts the wash 7 for the scope used for the examination 7 at 11:20, which had been placed in the queue for the washing apparatus 1.

As described, using two washing apparatuses and four scopes, all the examinations listed in the examination schedule shown in Fig. 3 can be performed without changing the start time of the examinations.

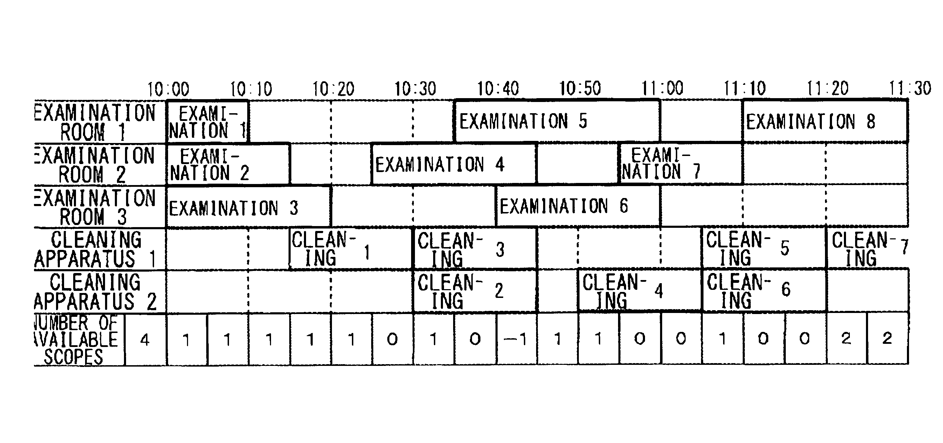

Fig. 4 is a view showing an example of the transition of both an examination schedule and the number of available scopes (prior to any change in the start time of the examination) according to the embodiment 1 (some delay in washing-start time). The examination schedule is the same as the examination schedule in Fig. 3. The difference between Fig. 3 and Fig. 4 lies in that a nurse or a worker in charge of the washing task does not set, in the washing apparatus 2, the scope used for the examination 2 at 10:20 but at 10:30 due to being busy or lazy. In other words, the difference minutes. This results in the negative number of scopes that are available at 10:30, at which the examination 6 should start. In other words, there is a shortage of one scope. Therefore, the examination 6 cannot start at 10:40.

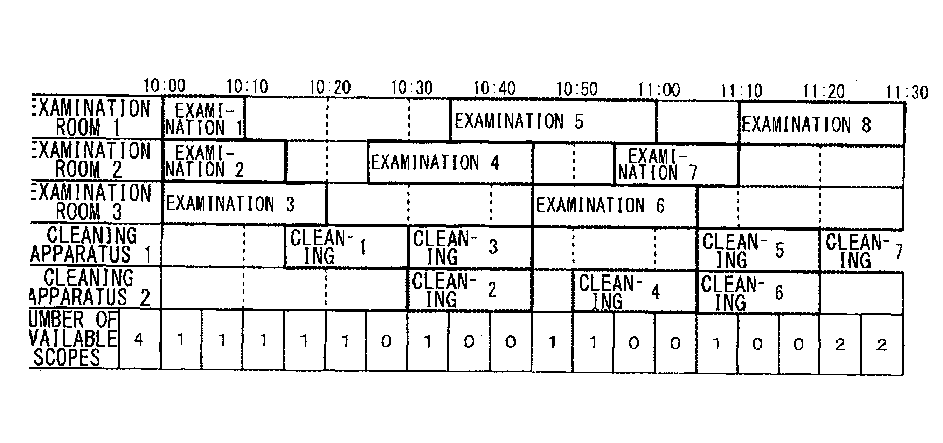

Fig. 5 is a view showing an example of the transition of both an examination schedule and the number of available scopes (after any change in the start time of the examination) according to the embodiment 1 (some delay in washing-start time). Since the start time of the wash 2 is delayed and is not 10:20 but 10:30, the completion time of the wash 2 becomes 10:45 instead of 10:35. As a result, the number of available scopes becomes zero between 10:30 and 10:45 and becomes one after 10:45. Therefore, the examination 6, which is scheduled from 10:40 to 11:00, cannot start at 10:40. Thus, the start time of the examination 6 needs to be changed to after 10:45, at which time the number of available scopes is changed from zero to one. In Fig. 5, the start time and the completion time of the examination 6 are changed to 10:45 and 11:05, respectively.

When a subsequent examination is scheduled in the examination room where the rescheduled examination is performed, the subsequent examination may be delayed by the same amount of time by which the rescheduled examination is delayed; and when the unoccupied time between the subsequent examination and the rescheduled examination is not less than time of the subsequent examination does not need to be changed.

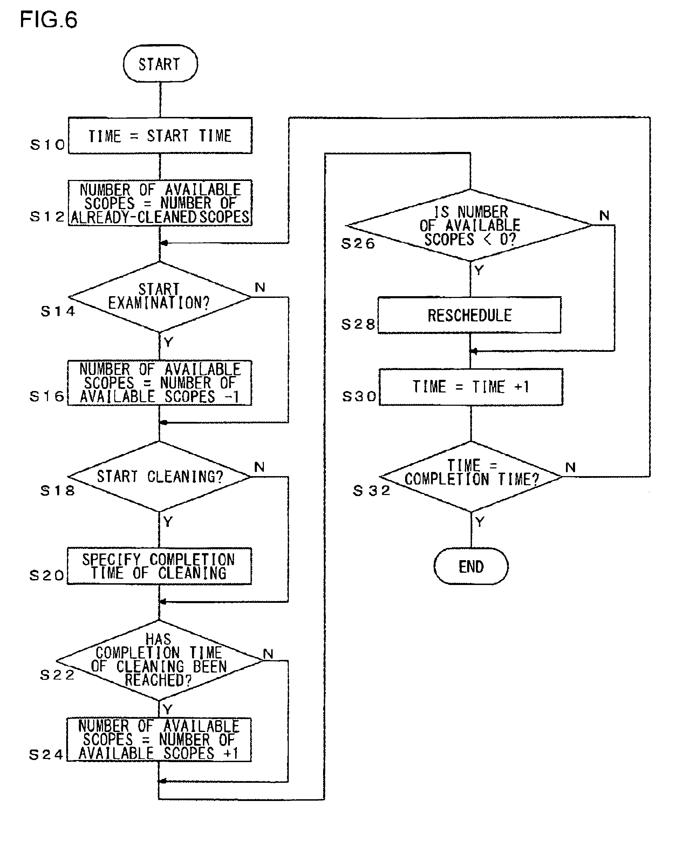

Fig. 6 is a flowchart showing a reschedule process of an examination management system 500 according to the embodiment 1. In the reschedule process, two parameters, i.e., "time" and "the number of available scopes," are used. Upon the start of the reschedule process, the start time of a simulation is set to be the "time" as an initial value setting (S10). The simulation may be set so as to be performed at a predetermined time interval (for example, an interval of five minutes). The number of already-washed scopes that are available at the start time of the simulation is also set to be "the number of available scopes" as an initial value setting (S12).

Referring to the examination schedule stored in the examination schedule memory unit 10, the rescheduling unit 30 determines whether or not there will be any examination that will start at the "time" (S14). When there is any examination (Y in S14), the value of "the number of available scopes" is decremented (S16). In Fig. 6, the value is decremented by one. However, when there are multiple examinations that start at the time indicated by the "time," the number of examinations is subtracted from the value of "the number of available scopes." When there is no examination that starts at the time indicated by the "time"

The rescheduling unit 30 determines whether or not, based on the washing-start notice acquired by the operation-status acquisition unit, there is any wash that starts at the "time" (S18). When there is a wash (Y in S18), the completion time of the wash is specified by adding the washing time of the washing apparatus to the start time of the wash (S20). When there is no wash that starts at the time indicated by the "time" (N in S18), the process of step S20 is skipped.

The rescheduling unit 30 determines whether or not the previously-specified washing-completion time has been reached (S22). When the washing-completion time has been reached (Y in S22), the value of "the number of available scopes" is incremented (S24). In Fig. 6, the value is incremented by one. However, when there are multiple washes that finish at the time indicated by the "time," the number of the washes is added to the value of "the number of available scopes." When the washing-completion time has not been reached at the "time" (N in S22), the process of step S24 is skipped.

The rescheduling unit 30 determines whether or not the value of "the number of available scopes" is below zero (S26). When the value is below zero (Y in S26), the rescheduling unit 30 reschedules the examination schedule so that the value of "the number of available scopes" does not examination at which the value of "the number of available scopes" is below zero is delayed until when the first wash performed after the start time is finished. When "the number of available scopes" is at least zero (N in S26), the process of step S28 is skipped.

The value of the "time" is incremented (S30). More specifically, one minute is added. The rescheduling unit 30 determines whether or not the value of the "time" has reached the completion time of the simulation (S32). The completion time of the simulation may be set to be the time obtained by adding a predetermined amount of time (for example, one hour) to the start time of the simulation. When the value of the "time" has not reached the completion time of the simulation (N in S32), the flow moves to the step S14, and continues the processes after the step S36. When the value of the "time" has reached (Y in S32), the above rescheduling process is terminated.

As described above, according to the embodiment 1, acquiring the operation status of a washing apparatus in real time and carrying out, at a predetermined interval, a simulation for predicting whether or not there will be shortage in scopes allow for the detection of the necessity of changing an examination schedule as early as possible and thus for an early change of the examination schedule.

Upon the change of the examination schedule, carried by a patient scheduled for the examination whose start time has been changed allows for the patient to be promptly notified of the change. Upon the change of the examination schedule, transmitting the changed examination schedule to the medical-staff terminal apparatus 300 allows for the medical staff to be promptly notified of the change.

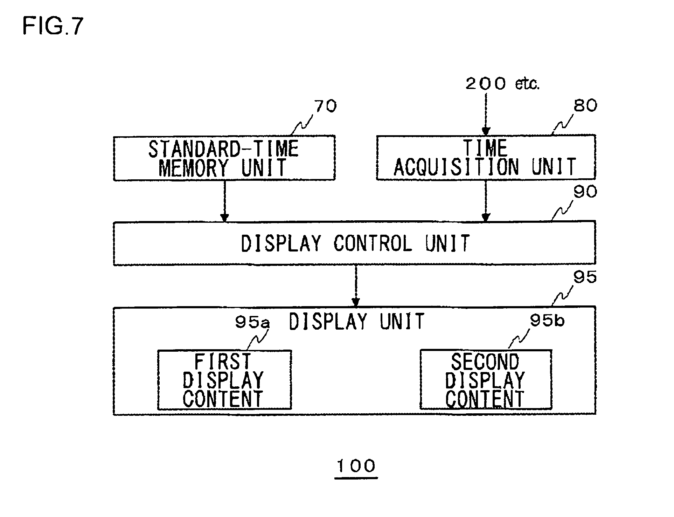

Fig. 7 is a functional block diagram showing the configuration of the examination management apparatus 100 according to the embodiment 2. The examination management apparatus 100 according to the embodiment 2 is provided with a standard-time memory unit 70, a time acquisition unit 80, and a display control unit 90. These components may be provided to the examination management apparatus 100 according to the embodiment 1. In Fig. 7, a display unit 95 is depicted as a component of the examination management apparatus 100. The display unit 95 may be a display unit exclusively for the examination management apparatus 100, a display unit provided to the endoscopic system 200, or a display unit provided to the medical-staff terminal apparatus 300.

The standard-time memory unit 70 stores, according to the examination time, the respective standard time of a plurality of processes that constitute one examination. The examination in the embodiment 2 is a broad-sense examination and a concept that indicates any process until the patient leaves the room. On the other hand, the examination in the embodiment 1 is a narrowly-defined examination and a concept that indicates any process performed from when a scope is inserted into the body of a patient until the scope is pulled out. The standard time of each process may be the time statistically obtained based on multiple sets of sample data or may be the time determined based on the knowledge of a doctor.

The time acquisition unit 80 acquires the start time and the completion time of each process of an examination from at least one device installed in the examination room where an examination is performed. In other words, even when there are multiple examination rooms, one device is necessary for an examination process. Such a device includes, for example, the endoscopic system 200, the medical-staff terminal apparatus 300, and a wide variety of sensors. For example, an endoscopic examination includes processes such as a "pretreatment," a "narrowly-defined examination," and a "rest."

There is a case where a patient takes GASCON (dimethicone) as pretreatment for an upper gastrointestinal tract endoscopic examination. There is a case where a patient takes an oral intestinal lavage preparation as a pretreatment for a lower gastrointestinal tract endoscopic examination. As the above device, a sensor may be provided water for taking GASCON or an oral intestinal lavage preparation. Water for taking GASCON or an oral intestinal lavage preparation is poured into the cup, and the time acquisition unit 80 then acquires, as the start time of the pretreatment, the time at which the sensor detects at least a predetermined weight. Alternatively, the time acquisition unit 80 acquires, as the start time of the pretreatment, the time at which the sensor detects a weight change from a predetermined weight to zero when a patient picks up a cup into which water for taking GASCON or an oral intestinal lavage preparation has been poured.

As the above device, a sensor that detects the insertion of a scope into the body of a patient may be provided. The time acquisition unit 80 acquires the time at which the sensor detects the insertion of a scope as the start time of the narrowly-defined examination. The time acquisition unit 80 acquires the time at which the sensor detects the pulling-out of a scope as the completion time of the narrowly-defined examination. As the above device, a sensor for detecting whether or not a patient is lying in bed may be provided. The time acquisition unit 80 acquires the time at which the sensor detects that a patient has left his/her bed as the completion time of the above "rest."

The time acquisition unit 80 may be notified of the start time and the completion time of each of the completion time of each of the processes through the medical-staff terminal apparatus 300 carried by a nurse or installed in an examination room. It may be designed so that the start time and the completion time of each of the processes, which are specified by various types of sensors or the like are transmitted to the examination management apparatus 100 via the network 400, or it may be designed so that the start time and the completion time are transmitted to the examination management apparatus 100 via the network 400 after they are once received by the endoscopic system 200 or the medical-staff terminal apparatus 300.

The display control unit 90 displays, on a display unit of the terminal apparatus installed in an examination room, a first display content 95a, which indicates the progress status to be achieved of an examination based on the standard time, which is based on the examination time, stored in the standard-time memory unit 70, and a second display content 95b, which indicates the actual progress status based on the start time and the completion time of each of the processes of the examination acquired by the time acquisition unit 80. Such a device includes the endoscopic system 200 and the medical-staff terminal apparatus 300. The display control unit 90 may display side-by-side the first display content 95a and the second display content 95b, with the time axes lined up. The display regarding processes from current to past and display the first display content 95a regarding future processes. When the actual progress status is in delay, the delay may be announced by changing a display color, using a flashing display, or further using sounds such as an alarm or beeps. Furthermore, when the progress is in delay, the respective standard time of the future processes may be shortened based on the time of delay.

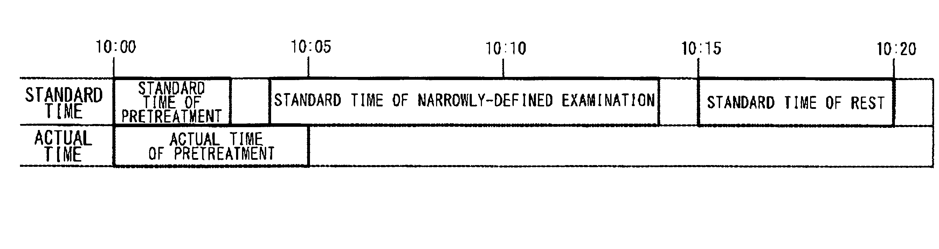

Fig. 8 is a diagram showing an example of a screen display-controlled by the display control unit 90 according to the embodiment 2 (in the middle of an examination). The broad-sense examination in this example includes, for example, a pretreatment process, a narrowly-defined examination process, and a rest process, and the periods of the standard time are three minutes, ten minutes, and five minutes, respectively. An unoccupied time of one minute is provided between processes. Furthermore, a subprocess obtained by subdividing the processes may be provided. For example, the pretreatment process may be subdivided into a treatment-water process and a rest process.

The display example in Fig. 8 displays, side-by-side, a standard-time display column 92 as the first display content 95a and a actual-time display column 94 as the second display content 95b, with the time axes lined up. Fig. 8 shows the screen at the point of 10:05. The pretreatment pretreatment is in delay. Checking the screen, the medical staff from doctors on down can be aware that the current process and subsequent processes need to be performed as rapidly as possible.

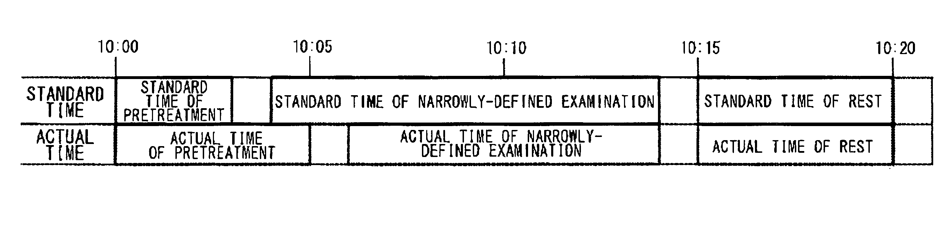

Fig. 9 is a diagram showing an example of a screen display-controlled by the display control unit 90 according to the embodiment 2 (after an examination). Having seen the screen shown in Fig. 8, a doctor promptly completed the narrowly-defined examination processes in eight minutes as opposed to the standard time of ten minutes. This allowed for the broad-sense examination to be completed as scheduled.

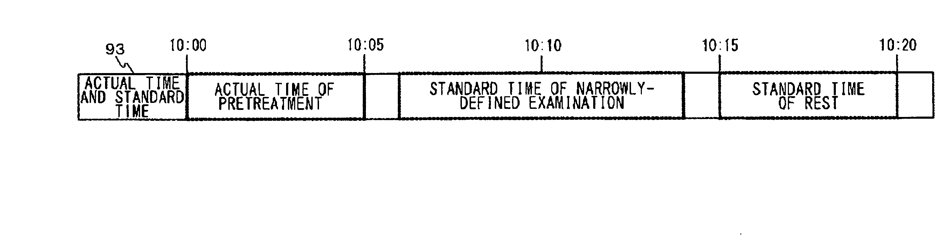

Fig. 10 is a diagram showing another example of a screen display-controlled by the display control unit 90 according to the embodiment 2. The screen displays the standard-time display column 92 and the actual-time display column 94 both in a display column 93. The actual time is displayed for the past with reference to the present, and the standard time is displayed for the future. When the process actually being performed at the present time is a process to be performed prior to the process that was supposed to be performed, the display control unit 90 shortens the standard time of the process that was supposed to be performed based on the delay in the process being performed and displays the shortened standard time. In other words, not the completion time but the standard time of the process that was supposed

Fig. 10 shows the screen at the point of 10:05. The narrowly-defined examination process should be started at 10:04; however, since the pretreatment process before the narrowly-defined examination process is in delay, the start time of the narrowly-defined examination process is being gradually put off. In Fig. 10, the start time is put off until 10:06. When the pretreatment process is further delayed, the start time of the narrowly-defined examination process is further put off. In the figure, an example is explained where the standard time of a process subsequent to a process in delay; however, the periods of the respective standard time of a plurality of processes after the process in delay may be shortened. In this case, each of the subsequent processes may be shortened by the amount of time obtained by dividing the time of delay by the number of the subsequent processes. When changing the standard time, the operating time of an examination room may be changed depending on the operating capacity of the examination room. In other words, the standard time may be changed based on the standard time for a special examination, the standard time based on the knowledge of a doctor, and the standard time statistically obtained according to examination details since it may take a long time for the "pretreatment," "narrowly-defined examination," or the like depending on the examination details performed in an examination room. washing apparatus that changes in chronological order, the respective standard time of the "pretreatment" and the "narrowly-defined examination" may be changed depending on the operation status of the examination room or on the examination status.

As described above, according to the embodiment 2, displaying the standard time and the actual time of each of the broad-sense examinations allows for the operational efficiency improvement to be supported for medical staff from doctors on down. Since medical staff can be made aware of, for example, the amount of time necessary for a rest, improvements in the safety of medical practices can be also supported.

Described above is an explanation based on the embodiments of the present invention. These embodiments are intended to be illustrative only, and it will be obvious to those skilled in the art that various modifications to constituting elements and processes could be developed and that such modifications are also within the scope of the present invention.

In order to simplify the explanation, the types of scopes are not taken into consideration in the embodiment 1. However, there are actually various types of scopes such as scopes used for upper gastrointestinal tracts, scopes used for lower gastrointestinal tracts, and scopes used for are used, the number of available scopes needs to be provided for each of the types, or an examination schedule needs to be created for each of the types.

In order to simplify the explanation, the explanation has been made based on the premise that each examination progresses in an ideal manner as indicated by the start time and completion time specified in an examination schedule in the embodiment 1. However, in reality, an examination does not often times start or end as scheduled. Desirably, the rescheduling unit 30 acquires the actual start time and completion time of each examination from, for example, the endoscopic system 200 and appropriately changes an examination schedule.

### [Explanation of Reference Numerals]

10 examination-schedule memory unit
20 operation-status acquisition unit
30 rescheduling unit
40 first notification unit
50 second notification unit
70 standard-time memory unit
80 time acquisition unit
90 display control unit
92 standard-time display column
94 actual-time display column
95a first display content
95b second display content
100 examination management apparatus
200 endoscopic system
210 washing apparatus
300 medical-staff terminal apparatus
400 network
500 examination management system

### [Industrial Applicability]

The present invention can be applied in a field where managing a schedule of examinations for which endoscopes are used.

## Claims

1. An examination management apparatus comprising:
an examination schedule memory unit operative to store an examination schedule including a plurality of examinations;
an operation-status acquisition unit operative to acquire operation-status that indicates the operation status of a washing apparatus for washing a scope used in the examination; and
a rescheduling unit operative to change the start time of at least one examination included in the examination schedule in accordance with the operation-status acquired by the operation-status acquisition unit.

2. The examination management apparatus according to Claim 1 wherein
the examination-schedule memory unit stores the start time and the completion time of each examination included in the examination schedule,
the operation-status acquisition unit acquires washing-start time for starting the washing of a used scope generated upon the completion of each examination by the washing apparatus, and
the rescheduling unit specifies the washing-completion acquired by the operation-status acquisition unit, determines whether or not an already-washed scope will be available at the start time of each examination based on the specified washing-completion time and the start time of each examination that is specified based on the examination schedule, and changes, when it is determined that there will be no already-washed scopes available, the start time of the examination to be after the time at which an already-washed scope will be available upon the completion of the washing.

3. The examination management apparatus according to Claim 2 wherein the rescheduling unit simulates, by using the number of already-washed scopes at the start of a simulation as an initial value, the transition of the number of available scopes in which the number decreases upon the start of each examination after the start time of the simulation and in which the number increases upon the completion of the washing a used scope, thereby predicting whether or not the already-washed scope will be available at the start time of each examination included in the examination schedule after the start time of the simulation and predicting, when the already-washed scope will not be available, the time at which the already-washed scope will become available.

4. The examination management apparatus according to notification unit operative to transmit schedule-change information, including at least the changed start time of an examination, to a patient terminal apparatus that can be referred to by a patient scheduled for the examination whose start time has been changed by the rescheduling unit.

5. The examination management apparatus according to any one of Claims 1 to 4 further comprising a second notification unit operative to transmit the examination schedule as changed to a patient terminal apparatus that can be referred to by medical staff when the start time of an examination is changed by the rescheduling unit.

6. The examination management apparatus according to any one of Claims 1 to 5 comprising:
a standard-time memory unit operative to store, according to examination time, the respective standard time of a plurality of processes that constitute one examination;
a time acquisition unit operative to acquire the start time and the completion time of each process of the examination from at least one device installed in the examination room where the examination is performed; and
a display control unit operative to display, on a display unit of the terminal apparatus installed in the examination room, a first display content, which indicates on the standard time that is based on the examination time, and a second display content, which indicates the actual progress status based on the start time and the completion time of each of the processes of the examination acquired by the time acquisition unit.
